# EUROPEAN PATENT APPLICATION

(11) **EP 1 985 239 A1**
(43) Date of publication of application: **29.10.2008**
(21) Application number: 08001708.0
(22) Date of filing: 30.01.2008
(51) Int. Cl.: A61B 10/06, A61B 17/32

(54) **Biopsy forceps**

(30) Priority: 24.04.2007 CN 200720036717 U
(71) Applicant: Micro-Tech (Nanjing) Co., Ltd., Nanjing Jiangsu 210061 (CN)
(72) Inventor: Derong, Leng, 210037 Nanjing (CN); Libao, Shen, 210045 Nanjing, Jiangsu (CN); Liang, Wang, 2254 Taixing, Jiangsu (CN)
(74) Representative: Paul, Dieter-Alfred

(57) **Abstract**

A biopsy forceps includes a pair of biopsy jaws (5, 6), wherein the distal ends of the jaws (5, 6) comprise opposed jaw cups (B, 9), each of which is provided with corresponding cutting elements (10, 11), and the proximal ends of the jaws (5, 6) are connected to an actuation arrangement for opening and closing the jaw cups (8, 9). The jaws (5, 6) are pivotably movable relative to each other, and the cutting elements (10, 11) are provided with sharp cutting edges (10a, 11a) and are off set in the inward-outward direction in such a way that during the closing movement of the jaws (5, 6) the cutting elements (10, 11) can pass each other, while their cutting edges (10a, 11a) come into contact with each other and are moved relative to one another keeping the contact to obtain a scissoring effect between them.

## Description

The present invention relates to a biopsy forceps including a pair of biopsy jaws, which are movably connected to each other, wherein the distal ends of the jaws comprise opposed jaws cups, each of which is provided with corresponding cutting elements, and the proximal ends of the jaws are connected to an actuating assembly for opening and closing the jaw cups including an elongate flexible tube and an actuating member extending through the tube.

At present endoscopy is widely used in clinics or surgery as a kind of diagnostic method. When making an endoscopic examination of a particular site on a patient's body, it is common to take at least one tissue sample from that site for analysis. Such endoscopic biopsy procedures are typically performed with the endoscope and an endoscopic biopsy forceps device (bioptome), which allows to take the tissue sample from the examined site in the patient's body with little pains and little ravage.

The endoscope is a long flexible tube carrying fiber optics and having a narrow lumen through which the bioptome can be inserted. During a biopsy tissue sampling operation the surgeon guides the endoscope to the biopsy site while viewing the biopsy site through the fiber optics of the endoscope. The bioptome is inserted through the narrow lumen of the endoscope until the opposed jaws thereof arrive at the biopsy site. While viewing the biopsy site through the fiber optics of the endoscope, the surgeon positions the jaws around a tissue to be sampled and manipulates the actuation means so that the jaws close around the tissue. A sample of the tissue is then cut and/or torn away from the biopsy side while it is trapped between the jaws of the bioptome. Keeping the jaws closed, the surgeon withdraws the bioptome from the endoscope and then opens the jaws to collect a biopsy tissue sample.

In general such biopsy forceps, as it is known from EP 817 593 B1, includes a long flexible coil having a pair of jaws at the distal end and manual actuating means at the proximal end for opening and closing the jaws. Specifically the actuation means comprises an elongate flexible coil and a drive wire extending therethrough. One end of the drive wire connects a linkage structure in the jaws and the other end of the drive wire connects with the movable part on the manual actuation means such as a handle. In order to open and close the jaws parts the handle is pushed and pulled in order to move the drive wire relative to the flexible tube and thus actuate the linkage structure.

In the previous endoscopic bioptome known from EP 0 817 593 B1 the jaws have opposed cutting edges, which in the fully closed position of the jaws come into full contact and basically pinch or nip off the doubtful tissue sample. However, when during sampling the operator pulls the drive wire to nip the tissue with unsufficient power, the tissue will be torn from the organ and with the result, that the patient may suffer pains. Also the operation is hard and inconvenient.

Accordingly it is the object of the present invention to provide a biopsy forceps of the initially mentioned kind allowing an easy handling and removal of tissue samples without causing pains for the patient.

According to the present invention this object is solved in that the jaws are pivotably movable relative to each other and that the cutting elements are provided with sharp cutting edges and are off set in the inward-outward direction in such a way that during the closing movement of the biopsy jaws the cutting elements can pass each other, while their cutting edges come into contact with each other and are moved relative to one another keeping the contact to obtain a scissoring effect between them.

According to the present invention the tissue samples are not pinched or nipped off by squeezing action between the cutting edges of the jaws. Instead provision is made, that a real scissor-like cutting action takes place between the cutting edges. Specifically when closing the jaws due to the hinge connection between the jaws the cutting edges come into contact firstly at their proximal ends closest to the pivot axis, and during the further closing process the contact points are continuously moved forwardly in the direction of the distal end. At the same time the rear parts of the cutting edges, which have already performed the cutting action are moved along the inner/outer surface of the respective other cutting element. Due to this real cutting action tissue samples can be easily taken and a full separation of the tissue sample can be guaranteed without the danger, that a part of the sample remains attached to the rest of the tissue and has to be torn away. Also, since there is only a point contact between the cutting edges, the necessary cutting forces are comparatively low and accordingly the biopsy forceps of the present invention is easy to handle. Moreover the biopsy forceps according to the present invention offers a simple and inexpensive arrangement free of complex linkages and multiple hinge points.

According to one embodiment of the present invention the cutting elements are pivotably joined to each other in their middle portions.

Moreover the cutting edges can be continuous or discontinuous. In the later case they may in particular be saw-tooth shaped.

Also the cutting elements are preferably U-shaped, wherein the ends of the legs of the U-form are positioned near the proximal ends of the jaw cups.

As to further features of the present invention reference is made to the subclaims as well as to exemplary embodiments of the invention, which are shown in the drawings, wherein
- Figure 1: shows one embodiment of a biopsy forceps according to the present invention,
- Figure 2: is a further embodiment of a biopsy forceps according to the present invention,
- Figure 3: is a perspective view showing a jaw arrangement for a biopsy forceps according to the present invention,
- Figure 4: is a perspective view of the upper jaw of the jaw arrangement of Figure 3 taken from below,
- Figure 5: is a perspective view of the lower jaw of the jaw arrangement of Figure 3 taken from above,
- Figure 6: is a schematic view of the jaws, when the cutting edges come into contact with each other,
- Figure 7: is a schematic view showing the jaws of Figure 6 when they are further closed,
- Figure 8: is a perspective view of a further jaw arrangement for a biopsy forceps according to the present invention,
- Figure 9: shows a perspective view of the upper jaw of the jaw arrangement of Figure 8, and
- Figure 10: shows a perspective view of the lower jaw of the jaw arrangement of Figure 8.

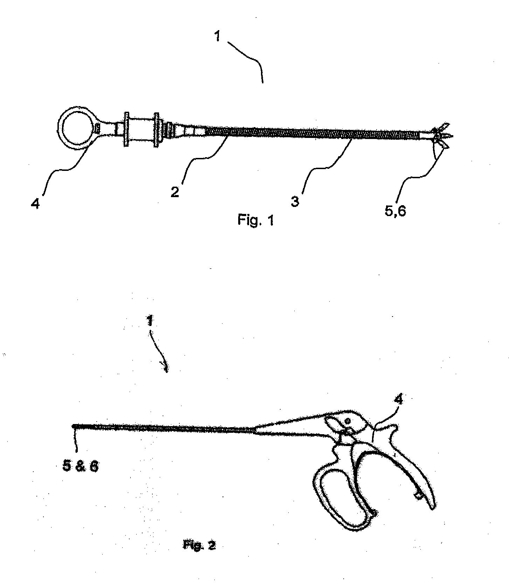

Figure 1 shows an embodiment of an endoscopic biopsy forceps 1 according to the present invention. This biopsy forceps 1 includes an actuation arrangement comprising an elongate flexible tubular catheter shaft 2 and a control wire 3, that extends through the catheter shaft 2 and is connected at its proximal end to an actuation means in the form of an actuation handle 4, by which the operator may pull or push on the wire. Alternatively the actuation means may be a pistol-handle, as it is depicted in Figure 2.

The biopsy forceps moreover comprises a pair of left and right jaws 5,6, which are connected in their middle parts to a pivot 7. The proximal ends of the jaws 5, 6 are connected with the actuation arrangement, here the control wire 3, and the distal ends form an opening structure with jaw cups 8, 9, that can be pivotably closed or opened by pushing or pulling the handle 4.

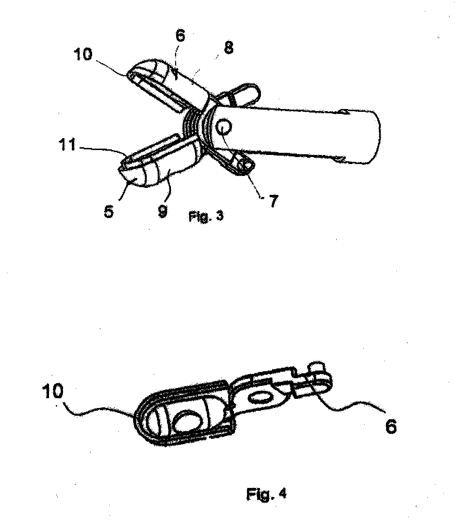

According to the present invention the jaw cups 8, 9 each carry a sharp rim or cutting element 19, 11, which have a general U-shape, wherein the ends of the U-legs are positioned at the proximal end of the jaw cups 8, 9. According to the present invention the cutting elements 11 are off set in the inward-outward direction in such a way, that the cutting elements 10, 11 can pass each other during the closing movement of the biopsy forceps 1, while at the same time their cutting edges 10a, 11a come into contact with each other and are moved relative to one another keeping the contact to obtain a scissoring effect between them. Specifically the U-shaped cutting element 10 of the right or upper jaw 6 shown in Figure 3 is positioned outwardly of the cutting element 11, which is provided on the left or lower jaw 5. This arrangement guarantees, that a real scissor-like cutting action takes place between the cutting edges 10a, 11a, which are here provided by right angled step surfaces of the cutting elements 10,11.

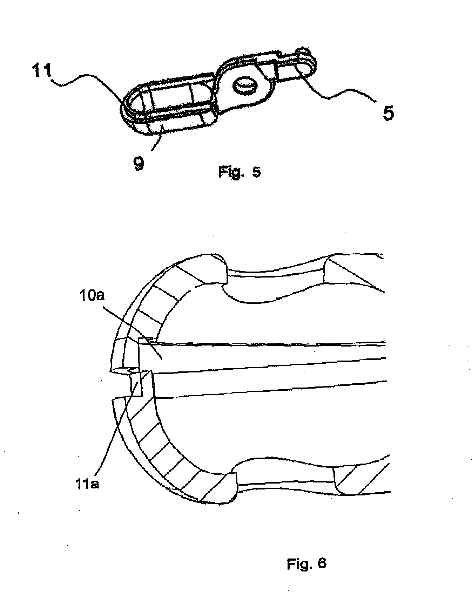

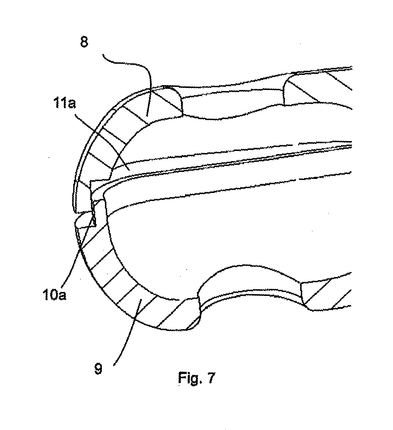

Specifically, when the jaws 5, 6 are closed, they are pivoted around the pivot 7 towards each other, so that the cutting edges 10a, 11a come into contact with each other firstly at their proximal ends, thus obtaining a shearing or cutting point (see Fig. 6). When the closing process is continued, the contact points at both legs of the cutting elements 10, 11 are continuously moved forwardly in the direction of the distal end. At the same time those parts of the cutting edges 10a, 11a on the proximal side of the cutting points, which have already performed the cutting action, are moved along the inner/outer surfaces of the respective other cutting element 10, 11 , with or without losing contact (see Fig. 7). The closing process is completed, when the cutting element 10 of the right or upper jaw 5 rests on the outer flat path 12 surrounding the cutting element 11 of the lower jaw 6.

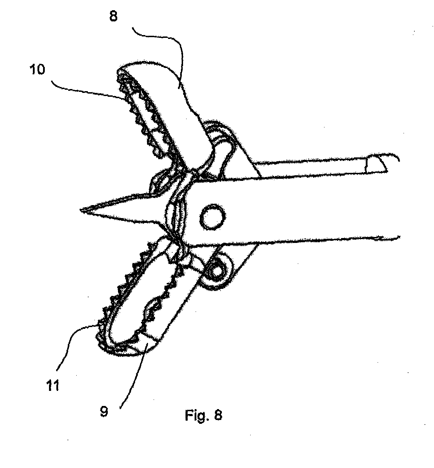

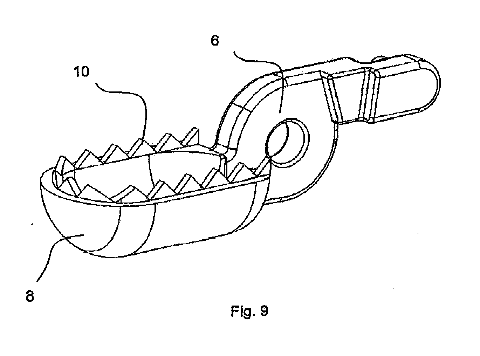

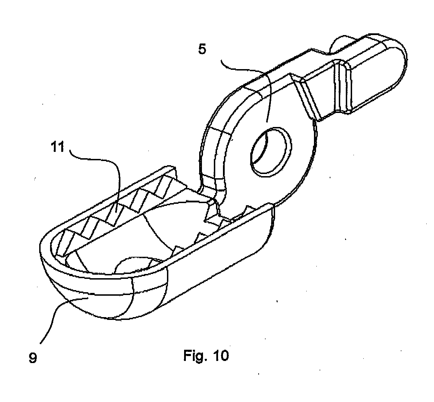

In the embodiment shown in Figures 3 to 7 the cutting elements have a continuous cutting edge 10a, 11a. This, however, is not a necessary feature. Instead the cutting edges 10a, 11a may be discontinuous and in particular obtain a saw-tooth form as it is depicted in Figures 8 to 10.

## Claims

1. Biopsy forceps including a pair of biopsy jaws (5, 6), which are movably connected to each other, wherein the distal ends of the jaws (5, 6) comprise opposed jaw cups (8, 9), each of which is provided with corresponding cutting elements (10, 11), and the proximal ends of the jaws (5, 6) are connected to an actuation arrangement for opening and closing the jaw cups (8, 9) including an elongate flexible tube (2) and an actuating member (3) extending through the tube, **characterized in that** the jaws (5, 6) are pivotably movable relative to each other and that the cutting elements (10, 11) are provided with sharp cutting edges (10a, 11a) and are off set in the inward-outward direction in such a way that during the closing movement of the jaws (5, 6) the cutting elements (10, 11) can pass each other, while their cutting edges (10a, 11a) come into contact with each other and are moved relative to one another keeping the contact to obtain a scissoring effect between them.

2. Biopsy forceps according to claim 1, **characterized in that** the cutting elements (10, 11) are pivotably joined to each other in their middle portions.

3. Biopsy forceps according to claim 1 or 2, **characterized in that** the cutting edges (10a, 11a) are continuous.

4. Biopsy forceps according to claim 1 or 2, **characterized in that** the cutting edges (10a, 11a) are discontinuous and in particular saw-tooth shaped.

5. Biopsy forceps according to any preceding claim, wherein the cutting elements (10, 11) are U-shaped, wherein the ends of the legs of the U-form are positioned near the proximal end of the jaw cups (8, 9).
